# EUROPÄISCHE PATENTANMELDUNG

(11) **EP 3 957 333 A1**
(43) Veröffentlichungstag der Anmeldung: **23.02.2022**
(21) Anmeldenummer: 21186117.4
(22) Anmeldetag: 16.07.2021
(51) Int. Cl.: A61L 2/00, A61L 2/10, A61L 2/24

(54) **UV-MODUL, SYSTEM UND VERFAHREN ZUR DEAKTIVIERUNG VON BIOLOGISCH KONTAMINIERTEN OBERFLÄCHEN IN EINEM INNERAUM EINES GERÄTS**

(30) Priorität: 19.08.2020 DE 102020121809; 21.08.2020 DE 102020121995
(71) Anmelder: Analytik Jena GmbH, 07745 Jena (DE)
(72) Erfinder: Moore, Thomas, 07751 Jena-Drackendorf (DE); Knippschild, Claus, 07745 Jena (DE); Hentschel, Martin, 07749 Jena (DE)
(74) Vertreter: Andres, Angelika Maria

(57) **Zusammenfassung**

Die Erfindung betrifft ein UV-Modul zur Deaktivierung von biologisch kontaminierten Oberflächen in einem Innenraum eines Geräts, umfassend:
- einen Träger,
- mindestens eine an dem Träger befestigte, zur Emission von UV-Strahlung ausgestaltete Strahlungsquelle, und
- eine mit der mindestens einen Strahlungsquelle verbundene Steuereinheit,
wobei die Steuereinheit dazu eingerichtet ist, mindestens eine Information zu empfangen und zu verarbeiten, um basierend auf der mindestens einen Information die Strahlungsquelle zur Emission von UV-Strahlung zu betreiben, insbesondere einzuschalten.

Die Erfindung betrifft außerdem ein System, das ein UV-Modul wie das zuvor beschriebene und ein Gerät umfasst, wobei das Gerät insbesondere zu Handhabung und/oder Untersuchung von biologisches Material umfassenden Proben dient, und einen Innenraum aufweist, in den das UV-Modul einbringbar ist.

Die Erfindung betrifft auch ein Verfahren zur Deaktivierung biologisch kontaminierten Oberflächen in einem Innenraum eines Geräts, insbesondere zur Handhabung und/oder Untersuchung von biologisches Material umfassenden Proben.

## Beschreibung

Die Erfindung betrifft ein UV-Modul zur Deaktivierung von biologisch kontaminierten Oberflächen in einem Innenraum eines Geräts, ein System mit einem Gerät und einem UV-Modul, das dazu dient, biologisch kontaminierte Oberflächen in einem Innenraum des Geräts zu deaktivieren und ein Verfahren zur Deaktivierung von biologisch kontaminierten Oberflächen in einem Innenraum eines Geräts, insbesondere zur Handhabung und/oder Untersuchung von biologisches Material umfassenden Proben.

In der Laboranalytik, insbesondere im Life-Science-Bereich und in der medizinischen Diagnostik, werden vielfach Proben untersucht, die biologisches Material umfassen. Hierbei werden Laborgeräte eingesetzt, die einen Innenraum, z.B. einen Probenraum, aufweisen, in den die Proben für eine Analyse oder für die Vorbereitung einer Analyse, z.B. für eine Vorbehandlung zur Zerkleinerung, Aufreinigung, Homogenisierung oder Anreicherung, positioniert werden. Beispiele für solche zur Aufnahme und Behandlung und/oder Analyse von Proben dienenden Innenräumen sind Waagen, Mühlen, Mörser, Pipettierautomaten, Thermocycler oder Real-Time-Thermocycler.

Die Proben werden häufig in Probengefäßen aus Plastikmaterial, z.B. einzelne Probenröhrchen oder sogenannten Wells von Mikro(titer)platten mit einer Vielzahl von Probengefäßen, gehandhabt und in einem Innenraum, z.B. einem Probenraum, in den Laborgeräten positioniert. Der Verschluss von solchen Probengefäßen erfolgt durch Kappen, Deckel und Klebefolien. Verschiedene Laborgeräte weisen mechanische Aufnahmen auf, die eine exakte Positionierung der eingesetzten Probengefäße erzwingen. Der Probenraum wird bei vielen Geräten manuell oder auch automatisch geschlossen, um eine Behandlung der Proben oder um Messungen ausführen zu können. Ein Thermocycler mit einem durch einen Deckel automatisiert verschließbaren Probenraum ist aus DE 10 2018 124 408 A1 oder DE 10 2018 124 412 A1 bekannt.

Viele der Arbeiten erfolgen manuell oder (teil-)automatisiert und es gibt keine aktive Kontrolle bezüglich der Dichtheit der Probengefäße. Somit besteht bei all diesen Arbeiten die Gefahr, dass Probenmaterial verschleppt wird und/oder Oberflächen in Innenräumen eingesetzter Geräte kontaminiert. Von besonderer Bedeutung sind dabei auch Aerosole, die über die Luft verwirbelt werden und sich in den Innenräumen absetzen. Die Innenräume sind für eine Reinigung oder Durchlüftung häufig nicht ausreichend zugänglich, so dass eine Wischdesinfektion schwierig durchführbar ist.

Zur Deaktivierung von biologisch, z.B. durch Pilze, Bakterien und Viren, kontaminierten Oberflächen oder auch von kontaminierten Medien, wie z.B. Wasser, wird konventionell UV-Strahlung eingesetzt. Die UV-Strahlung erzeugt in den Nukleinsäuren Modifikationen des originalen Bindungszustands verschiedener Molekülbausteine. Durch Bestrahlung mit UV-Strahlung kann daher der Zellkern von Mikroorganismen so verändert werden, dass eine Zellteilung nicht mehr möglich ist, bzw. dass Viren Zellen nicht mehr umprogrammieren können. In der Folge können sich die Mikroorganismen oder Viren nicht mehr vermehren bzw. vermehrt werden. Auch im Laborbetrieb wird UV-Strahlung zur Deaktivierung von kontaminierten Geräten, Flächen oder Volumina eingesetzt. Die Anmelderin bietet beispielsweise Cabinets und Workstations zur Probenvorbereitung für die Polymerasekettenreaktion (PCR) an, in denen mittels einer Kombination von Filtern und UV-Strahlung die Gefahr der Probenverschleppung erheblich reduziert werden kann. Die UV-Quelle ist innerhalb des Cabinets oder der Workstation angeordnet und kann zur Dekontaminierung des von dem Cabinet oder der Workstation eingeschlossenen Innenraums eingesetzt werden. Um ein versehentliches Bestrahlen der Proben zu vermeiden, dient zum Einschalten der UV-Quelle ein Schlüssel. Die in diesen Geräten verwendete UV-Quelle besteht aus einer oder mehreren im Deckenbereich des Cabinets oder der Workstation angeordneten UV-Röhren.

Auch ganze Laborräume werden, z.B. nachts, mit UV-C-Strahlung ausgeleuchtet und deren Luft durch Filtersysteme zirkuliert, die auch intern mit zusätzlichen UVC-Strahlungsquellen ausgerüstet sein können. Ein wesentliches praktisches Problem ist dabei die optische Abschattung und/oder die zu geringe Durchlüftung von Flächen oder Volumina. Insbesondere Oberflächen in Geräte-Innenräumen von Laborgeräten, wie z.B. Thermocyclern, Laborwaagen, Extraktionsautomaten oder nicht begehbaren Roboteranlagen können mit diesen Mitteln nicht mit ausreichender Sicherheit erreicht und vorhandene biologische Verunreinigungen deaktiviert werden.

Wegen der die DNA oder RNA von Zellen oder Viren verändernden Wirkung von UV-C-Strahlung, erfordert die Verwendung solcher Strahlung zur Dekontamination im Prozess- oder Laborbetrieb zusätzliche Schutzmaßnahmen, um Personen, die mit den entsprechenden Geräten umgehen, nicht zu gefährden.

Die Anmelderin bietet zur Dekontaminierung des von der Anmelderin hergestellten und angebotenen Extraktionsautomaten InnuPure C16 ein Dekontaminationsmodul an, das eine UV-Lampe umfasst. Das Dekontaminationsmodul kann in einer Lade des Extraktionsautomaten, die zur Aufnahme von Proben bestimmt ist, eingesetzt und in den zu dekontaminierenden Probenraum eingeschoben werden. Ist das Dekontaminationsmodul korrekt im Extraktionsautomaten positioniert, liegt ein Sicherheitsschalter an der Gerätewand so an, dass er den Stromkreis für die Stromversorgung der UV-Lampe schließt. Zusätzlich muss zum Einschalten der UV-Lampe ein weiterer Taster betätigt werden. Auf diese Weise wird ein unbeabsichtigtes Einschalten der UV-Lampe, insbesondere bei noch nicht korrekter Positionierung des Moduls im Gerät, verhindert.

Es ist die Aufgabe der vorliegenden Erfindung, eine weiter verbesserte Vorrichtung und ein Verfahren zur sicheren Deaktivierung von biologisch kontaminierten Oberflächen in Geräteinnenräumen anzugeben.

Diese Aufgabe wird erfindungsgemäß gelöst durch das UV-Modul gemäß Anspruch 1, das System gemäß Anspruch 5 und das Verfahren gemäß Anspruch 16. Vorteilhafte Ausgestaltungen sind in den abhängigen Ansprüchen angegeben.

Das erfindungsgemäße UV-Modul zur Deaktivierung von biologisch kontaminierten Oberflächen in einem Innenraum eines Geräts, umfasst:
- einen Träger,
- mindestens eine an dem Träger befestigte, zur Emission von UV-Strahlung ausgestaltete Strahlungsquelle, und
- eine mit der mindestens einen Strahlungsquelle verbundene Steuereinheit,
wobei die Steuereinheit dazu eingerichtet ist, mindestens eine Information zu empfangen und zu verarbeiten, um basierend auf der mindestens einen Information die Strahlungsquelle zur Emission von UV-Strahlung zu betreiben, insbesondere einzuschalten.

Das UV-Modul kann dazu eingerichtet sein, in den Innenraum eines Geräts eingebracht zu werden, um kontaminierte Oberflächen in dem Innenraum zu deaktivieren. Unter der Deaktivierung biologisch kontaminierter Oberflächen wird hier und im Folgenden die eingangs beschriebene Unschädlichmachung von Mikroorganismen, z.B. Pilzen, Bakterien, Algen, oder von Viren, die auf den Oberflächen vorliegen, verstanden. Die Unschädlichmachung mittels UV-Strahlung basiert auf der bereits erwähnten Veränderung der DNA oder RNA, die dazu führt, dass sich die Mikroorganismen oder Viren nicht mehr vermehren bzw. vermehren lassen können. Dies wird umgangssprachlich auch als Dekontamination bezeichnet. Der Fachbegriff der Dekontamination biologisch kontaminierter Flächen schließt jedoch häufig auch das Entfernen der Kontamination durch Reinigungsmaßnahmen, z.B. durch Wischdesinfektion, ein. Die hier beschriebene Deaktivierung oder Dekontamination eines Innenraums in einem Gerät mittels eines UV-Moduls kann eine solche weitere Reinigung umfassen, muss es jedoch nicht.

Die Steuereinheit kann beispielsweise eine elektrische oder elektronische Schaltung sein, die eine zur Verarbeitung der Information eingerichtete Logikeinheit aufweist. Die Logikeinheit kann eine Logikschaltung, z.B. auf Basis der Booleschen Algebra, oder eine komplexere Recheneinheit, z.B. einen Mikroprozessor, umfassen. Indem die Steuereinheit die Strahlungsquelle basierend auf der mindestens einen Information betreibt, z.B. um die Strahlungsquelle zur Emission von UV-Strahlung einzuschalten bzw. zur Emission von UV-Strahlung anzuregen, ist es möglich, den unautorisierten oder unbeabsichtigten Betrieb der mindestens einen Strahlungsquelle sicher zu unterbinden. Während die Funktion eines Sicherheitsschalters, wie er bei dem eingangs beschriebenen Dekontaminationsmodul nach dem Stand der Technik vorgesehen ist, durch Missbrauch umgangen werden kann, kann die Übermittlung einer Information an die Steuereinheit, auf der basierend die Steuereinheit die Strahlungsquelle betreibt, dazu dienen das Einschalten der Strahlungsquelle ausschließlich dann zu ermöglichen, wenn der Betrieb der Strahlungsquelle hinreichend sicher oder aufgrund bestimmter Vorgaben autorisiert ist. Ein missbräuchlicher oder unabsichtlicher Eingriff in die Übermittlung der Information an die Steuereinheit kann leichter unterbunden werden als die Umgehung eines mechanischen Sicherheitsschalters.

In einer vorteilhaften Ausgestaltung kann die mindestens eine Strahlungsquelle eine UV-LED umfassen, die dazu eingerichtet ist, Strahlung im UV-B- oder UV-C-Spektralbereich zu emittieren. Dabei wird unter dem UV-B-Spektralbereich der Spektralbereich zwischen 315 nm und 280 nm verstanden, unter dem UV-C-Spektralbereich der Spektralbereich zwischen 280 nm und 100 nm. Vorteilhaft emittiert die UV-LED Strahlung einer oder mehrerer Wellenlängen im Bereich zwischen 380 und 100 nm, bevorzugt zwischen 310 und 200 nm, besonders bevorzugt zwischen 280 und 250 nm, z.B. bei 278 nm mit ca. 10 nm Halbwertsbreite. Das UV-Modul kann in einer vorteilhaften Ausgestaltung mehrere, insbesondere eine Vielzahl, UV-LEDs aufweisen. Diese können vorteilhaft so auf dem Träger des UV-Moduls angeordnet sein, dass sie in mehrere verschiedene Raumrichtungen abstrahlen, um so einen Innenraum eines Geräts, in dem das UV-Modul eingesetzt ist, im Wesentlichen vollständig und ohne Abschattungen mit einer Strahlungsdosis, die zur Deaktivierung ausreicht, auszuleuchten. Die zur Deaktivierung von Mikroorganismen oder Viren in der Regel im Stand der Technik eingesetzten quecksilberhaltige Leuchten sind dagegen aus Umwelt- und Arbeitsschutzgründen kritisch einzuschätzen. Auch sehen mittlerweile Gesetze und Richtlinien in vielen Ländern vor, Quecksilber in Prozess- und Laborgeräten zu vermeiden. Ein Beispiel hierfür ist die RoHS-Richtlinie der EU. Im Unterschied zu solchen klassischen UV-Lichtquellen sind UV-LEDs kompakter, energiesparend, sehr flexibel einsetzbar, und sie benötigen keine lange Aufwärmphase.

Die mindestens eine Information, basierend auf der die Steuereinheit die Strahlungsquelle betreibt, z.B. einschaltet, kann von einem Gerät, in dem das UV-Modul eingesetzt wird, an die Steuereinheit übermittelt werden. So kann das Gerät mittels der an das UV-Modul übermittelten Information an die Steuereinheit signalisieren, dass das Einschalten oder Betreiben der mindestens einen Strahlungsquelle sicher ist. Das Gerät kann beispielsweise dazu eingerichtet sein, die mindestens eine Information dann zu übermitteln, wenn sich das Gerät in einem für das Einschalten oder Betreiben der mindestens einen Strahlungsquelle in einem vorgegeben, z.B. einem sicheren, Betriebszustand befindet. Ein solcher sicherer Betriebszustand kann z.B. dann vorliegen, wenn der Innenraum, in dem das UV-Modul zur Deaktivierung dort vorliegender biologischer Schadsubstanzen, z.B. Mikroorganismen oder Viren, angeordnet ist, vollständig gegenüber der Umgebung verschlossen ist, so dass keine schädliche bzw. für Anwender oder Laborpersonal gefährliche UV-Strahlung aus dem Innenraum nach außen dringen kann.

In einer weiteren vorteilhaften Ausgestaltung umfasst das UV-Modul mindestens einen Sensor, insbesondere einen Strahlungsdetektor, einen Bewegungssensor, einen Positionssensor, eine Kamera oder einen Temperatursensor, wobei der Sensor mit der Steuereinheit verbunden ist, um dieser ein Messsignal als die mindestens eine Information zu übertragen. Beispielsweise kann der Sensor dazu dienen festzustellen, ob sich das Gerät in dem für das Einschalten oder Betreiben der mindestens einen Strahlungsquelle sicheren Betriebszustand befindet und/oder ob das UV-Modul korrekt innerhalb des zu dekontaminierenden Innenraums angeordnet ist. Ist das UV-Modul beispielsweise dazu bestimmt, einen Innenraum eins Geräts zu dekontaminieren, der lichtdicht ausgestaltet ist, z.B. einem Innenraum eines Thermocyclers oder eines qPCR-Thermocyclers, in dem bestimmungsgemäß optische Messungen durchgeführt werden, kann der Sensor einen Strahlungsdetektor oder einen Tageslichtsensor umfassen. Anhand des Sensorsignals lässt sich in diesem Fall, beispielsweise mittels eines Soll- oder Schwellenwertvergleichs, ermitteln, ob der Innenraum, in dem sich das UV-Modul befindet, verschlossen, und somit dunkel ist. Die Steuereinheit des UV-Moduls kann beispielsweise dazu eingerichtet sein, einen Schwellenwertvergleich des Sensorsignals mit einem vorgegebenen Schwellenwert, der dem Sensorsignal bei verschlossenem Innenraum entspricht, durchzuführen, und abhängig von dem Vergleich die mindestens eine Strahlungsquelle einschalten.

In einer weiteren vorteilhaften Ausgestaltung kann das UV-Modul mindestens ein Temperierelement, z.B. mindestens ein Peltier-Element, aufweisen, das beispielsweise an dem Träger befestigt sein kann. Das mindestens eine Temperierelement kann zum Heizen und/oder Kühlen ausgestaltet sein. Bezüglich der mindestens einen Strahlungsquelle kann es so angeordnet sein, dass es die Strahlungsquelle im Betrieb kühlen kann. Der Betrieb des mindestens einen Kühlelements kann durch die Steuereinheit des UV-Moduls steuerbar sein.

Zur Abführung von Wärme der mindestens einen Strahlungsquelle kann das UV-Modul in einer alternativen Ausgestaltung Wärmekontaktflächen aufweisen, die an eine Temperiervorrichtung des Geräts, in dem das UV-Modul eingesetzt werden soll, angelegt werden können. Ein so ausgestaltetes UV-Modul kann beispielsweise in einem Probenraum eines Thermocyclers eingesetzt werden, derart, dass die Wärmekontaktflächen gegen einen Temperierblock des Thermocyclers, der im PCR-Betrieb zur Temperierung der Proben vorgesehen ist, anliegen.

Das UV-Modul kann in einer weiteren Ausgestaltung einen, insbesondere berührungslos aufladbaren, Akkumulator aufweisen, der mit der Steuereinheit und/oder dem mindestens einen Sensor als Spannungsquelle verbunden ist. Die vom Akkumulator zur Verfügung gestellte Energie kann zum Betrieb der Steuereinheit, des optional vorhandenen mindestens einen Sensor und/oder der Strahlungsquelle dienen. Alternativ kann das UV-Modul mittels eines Kabels oder einer flexiblen Leiterkarte mit dem Gerät verbindbar sein, um von diesem mit Energie versorgt zu werden. Das UV-Modul kann auch dazu eingerichtet sein, kontaktlos, beispielsweise über eine induktive Schnittstelle, von einer externen Energieversorgung, z.B. dem Gerät, mit Energie versorgt zu werden. Hierzu kann es eine zum Energieempfang eingerichtete Spule aufweisen, die mit den mit Energie zu versorgenden Komponenten, z.B. der mindestens einen Strahlungsquelle, der Steuereinheit und dem mindestens einen Sensor, verbunden ist.

In einer weiteren Ausgestaltung kann die Steuereinheit eine Schnittstelle zur kontaktlosen Kommunikation, insbesondere nach einem Bluetooth-, NFC-, RFID- oder einem Funk-Standard, oder Mittel zur optischen Kommunikation, z.B. eine modulierbare Lichtquelle und einen Lichtempfänger, aufweisen. Diese Schnittstelle kann zum Empfang der mindestens einen Information dienen.

In einer weiteren Ausgestaltung kann das UV-Modul einen Antrieb, z.B. einen Motor, umfassen, der insbesondere von der Steuereinheit steuerbar ist. Der Antrieb kann zur Bereitstellung mechanischer Funktionen innerhalb eines Innenraums eines Geräts dienen, z.B. um ein Verbrauchsmodul (englischer Fachbegriff: Consumable) innerhalb des Innenraums zu manipulieren, z.B. zu bewegen, zu entriegeln oder aus einer Ursprungslage herauszuheben. Hierzu kann das UV-Modul einen mit dem Antrieb verbundenen mechanischen Manipulator aufweisen, z.B. einen Stößel oder einen Kipp-Mechanismus. Der Motor kann von dem bereits erwähnten Akkumulator des UV-Moduls oder über eine an das UV-Modul anschließbare separate Energiequelle, z.B. eine Energieversorgung des Geräts, in dem das UV-Modul eingesetzt wird, mit Energie versorgt werden.

Die Erfindung umfasst auch ein System, das mindestens folgende Komponenten aufweist:
- ein Gerät, insbesondere zur Handhabung und/oder Untersuchung von biologisches Material umfassenden Proben, mit einem Innenraum; und
- ein in den Innenraum einbringbares UV-Modul nach einer der voranstehend beschriebenen Ausgestaltungen zur Deaktivierung von biologisch kontaminierten Oberflächen in dem Innenraum.

Das Gerät kann in einer vorteilhaften Ausgestaltung eine Logikeinheit aufweisen, die dazu eingerichtet ist, die mindestens eine Information an die Steuereinheit, beispielsweise in Form eines Signals, insbesondere eines Digitalsignals, zu übertragen. Die Logikeinheit kann eine Logikschaltung, z.B. auf Basis der Booleschen Algebra, oder eine komplexere Recheneinheit umfassen, die sowohl für die Ausführung logischer als auch arithmetischer Operationen geeignet ist. Die Recheneinheit kann z.B. einen Mikroprozessor umfassen. Die mindestens eine von der Logikeinheit an die Steuereinheit übermittelte Information kann ein Steuerbefehl sein, den die Steuereinheit durch Einschalten der mindestens einen Strahlungsquelle ausführt. Die mindestens eine Information kann auch ein Messwert oder eine aus einem Messwert abgeleitete Information, eine Kennung, eine Security-ID, ein Passwort, eine Authentisierungs-Information oder ein Security-Token sein. Beispielsweise kann die Logikeinheit dazu eingerichtet sein, eine bestimmte Kennung oder eine Authentisierungs-Information oder einen Token nur an die Steuereinheit des UV-Moduls zu übermitteln, wenn sich das Gerät in einem vorgegebenen Betriebszustand befindet. Somit kann die Authentisierungs-Information oder Kennung gleichzeitig zur Authentifizierung des Geräts und zur Überprüfung bzw. Sicherstellung des Vorliegens des vorgegebenen Betriebszustands durch die Steuereinheit des UV-Moduls dienen.

Die mindestens eine Information kann mehrere einzelne Informationen umfassen, z.B. kann die Logikeinheit eine Authentisierungs-Information, z.B. eine Kennung, einen Token oder ein Passwort, und zusätzlich einen Steuerbefehl senden, wobei die Steuereinheit die Logikeinheit als Quelle des Steuerbefehls zunächst anhand der Authentisierungs-Information authentifiziert und erst nach erfolgreicher Authentifizierung den Steuerbefehl ausführt.

Die Logikeinheit kann in einer weiteren Ausgestaltung dazu eingerichtet sein, die mindestens eine Information an die Steuereinheit zu übertragen, wenn sich das Gerät in einem vorgegebenen, insbesondere sicheren, Betriebszustand befindet. Der vorgegebene Betriebszustand des Geräts kann z.B. ein für die Deaktivierung mittels UV-Strahlung sicherer Zustand sein, in dem der zu dekontaminierende Innenraum des Geräts verschlossen ist, derart, dass keine schädliche UV-Strahlung aus dem Innenraum nach außen dringen kann. Der Betriebszustand kann auch ein dezidierter Reinigungs- bzw. Dekontaminations-Betriebszustand des Geräts sein. Beinhaltet der vorgegebene Betriebszustand beispielsweise, dass eine Abdeckung, z.B. eine Tür oder ein Deckel, des Innenraums vollständig geschlossen ist, so kann die Logikeinheit das Vorliegen des Betriebszustands anhand eines Signals eines Abdeckungs-Sensors, z.B. eines Mikrotasters, eines Bewegungssensors oder eines Näherungsschalters, ermitteln, der das Schließen der Abdeckung detektiert, und basierend auf dem Signal des Abdeckungs-Sensors die mindestens eine Information an die Steuereinheit ausgeben. Alternativ kann die Logikeinheit das Vorliegen des Betriebszustands anhand interner Daten des Geräts, z.B. anhand von Steuerinformationen einer Abdeckungs-Schließautomatik, ermitteln.

In einer weiteren Ausgestaltung können die Logikeinheit und die Steuereinheit zur drahtlosen Kommunikation, insbesondere nach einem Bluetooth-, NFC-, RFID-Standard, mittels Funk oder mittels einer optischen Kommunikation, miteinander verbunden sein. Die Logikeinheit und die Steuereinheit können auch zur leitungsgebundenen Kommunikation, beispielsweise durch Kabel oder eine flexible Leiterkarte und einen Steckverbinder, miteinander verbunden sein.

Die Steuereinheit kann dazu eingerichtet sein, die Logikeinheit anhand der mindestens einen Information zu authentifizieren, und erst nach erfolgreicher Authentifizierung die Strahlungsquelle zur Emission von UV-Strahlung einzuschalten. Dies kann beispielsweise mittels eines Hardware- oder Software-Tokens geschehen. Es ist möglich, dass die Logikeinheit die Information nur an die Steuereinheit übermittelt, wenn das Gerät in einem für die Deaktivierung des Innenraums mittels UV-Strahlung sicheren Zustand ist, z.B. wenn der Innenraum vollständig geschlossen ist, derart, dass keine UV-Strahlung nach außen dringt.

Die Logikeinheit kann dazu eingerichtet sein, die Steuereinheit zu authentifizieren, und erst nach erfolgreicher Authentifizierung die mindestens eine Information an die Steuereinheit zu übertragen. Dies kann beispielsweise mittels eines Hardware- oder Software-Tokens geschehen.

Der Innenraum kann dazu ausgestaltet sein, nach dem Einbringen oder durch das Einbringen des UV-Moduls verschlossen zu werden, derart dass keine UV-Strahlung aus dem Innenraum nach außen dringen kann. Beispielsweise kann das Gerät einen den Innenraum nach dem Einbringen des UV-Moduls, insbesondere lichtdicht, verschließende Abdeckung, z.B. eine Tür oder einen Deckel, aufweisen, die nach dem Einbringen des UV-Moduls verschließbar ist. Alternativ kann das UV-Modul in den Innenraum derart einbringbar sein, dass eine Seitenwand des UV-Moduls eine lichtdichte Abdeckung des Innenraums bewirkt.

In einer weiteren Ausgestaltung kann das System einen, insbesondere an dem UV-Modul angeordneten, Sensor umfassen, der dazu eingerichtet ist, ein Messsignal bereitzustellen, anhand dessen ermittelbar ist, ob sich das Gerät in dem für das Einschalten oder Betreiben der mindestens einen Strahlungsquelle sicheren Betriebszustand befindet und/oder ob das UV-Modul korrekt innerhalb des zu dekontaminierenden Innenraums angeordnet ist. Beispielsweise kann anhand des Messsignals ermittelbar sein, ob der Innenraum verschlossen ist. Dieser Sensor kann z.B. ein Lichtsensor, der bereits erwähnte Abdeckungssensor, eine Kamera oder eine Lichtschranke sein. Ist der Sensor an dem UV-Modul angeordnet, kann er neben der von dem Gerät bzw. der Logikeinheit des Geräts übermittelten mindestens einen Information eine zusätzliche, insbesondere redundante, Information für die Steuereinheit bereitstellen, anhand derer die Steuereinheit die Strahlungsquelle betreibt, z.B. einschaltet. Die Steuereinheit kann beispielsweise dazu eingerichtet sein, nur dann die mindestens eine Strahlungsquelle zur Emission von UV-Strahlung einzuschalten, wenn sie von der Logikeinheit des Geräts eine erste Information erhält, die einen vorgegebenen, insbesondere sicheren, Betriebszustand des Geräts, z.B. den verschlossenen Zustand des Innenraums, signalisiert, und wenn sie zusätzlich von dem mindestens einen Sensor eine zweite Information erhält, die den vorgegebenen, insbesondere sicheren, Betriebszustand des Geräts signalisiert.

In einer weiteren Ausgestaltung kann die mindestens eine Information, zu deren Empfang und Verarbeitung die Steuereinheit eingerichtet ist, um darauf basierend die Strahlungsquelle zur Emission von UV-Strahlung einzuschalten, das Messsignal dieses Sensors oder eine aus dem Messsignal abgeleitete Information umfassen. Beispielsweise kann die Steuereinheit das Messsignal des Sensors erhalten, um daraus abzuleiten, ob der Innenraum vollständig, insbesondere lichtdicht, verschlossen ist. Hierzu kann die Steuereinheit dazu eingerichtet sein, einen Vergleich des Messsignals oder eines daraus abgeleiteten Werts mit einem Soll-Signal oder Soll-Wert bzw. einem Schwellenwert durchzuführen, das bzw. der den vorgegebenen, insbesondere sicheren, Betriebszustand des Geräts, z.B. den vollständig geschlossenen Zustand des Innenraums, repräsentiert. In dieser Ausgestaltung muss die Steuereinheit nicht notwendigerweise ein Signal von der Logikeinheit des Geräts erhalten, um die Strahlungsquelle zur Emission von UV-Strahlung einzuschalten. Vielmehr kann sie dazu eingerichtet sein, die Strahlungsquelle allein basierend auf dem Messsignal des Sensors zu steuern. Eine höhere Betriebssicherheit wird jedoch erreicht, wenn die Steuereinheit auch von der Logikeinheit des Geräts eine Information erhält, anhand derer sie die Strahlungsquelle betreibt.

In einer weiteren Ausgestaltung kann die Logikeinheit des Geräts das Messsignal des mindestens einen Sensors erhalten und auswerten. Die Logikeinheit kann beispielsweise dazu eingerichtet sein, anhand des Sensorsignals zu ermitteln, ob das Gerät in einem vorgegebenen, z.B. für die Deaktivierung des Innenraums mittels UV-Strahlung sicheren, Betriebszustand ist, z.B. ob der Innenraum vollständig geschlossen ist, derart, dass keine UV-Strahlung nach außen dringt. Hierzu kann die Logikeinheit dazu eingerichtet sein, das Sensorsignal oder einen daraus abgeleiteten Wert mit einem den sicheren Zustand repräsentierenden Sollsignal oder einem Soll- oder Schwellenwert zu vergleichen. Die Logikeinheit kann weiter dazu eingerichtet sein, die mindestens eine Information an die Steuereinheit zu senden, basierend auf der die Steuereinheit die Strahlungsquelle betreibt, insbesondere einschaltet, wenn das Sensorsignal oder der daraus abgeleitete Wert dem vorgegebenen Betriebszustand, z.B. dem sicheren Betriebszustand des Geräts, entspricht. Um eine höhere Sicherheit zu gewährleisten, kann mindestens ein weiterer, insbesondere redundanter, Sensor im Gerät und/oder am UV-Modul vorgesehen sein, der der Logikeinheit und/oder der Steuereinheit ein weiteres Messsignal zur Verfügung stellt, aus dem ableitbar ist, ob das Gerät in einem für die Deaktivierung des Innenraums mittels UV-Strahlung sicheren Betriebszustand ist.

Das UV-Modul kann einen Temperatursensor und/oder einen Beschleunigungssensor und/oder einen Positionssensor und/oder eine Kamera aufweisen, wobei die mindestens eine Information, zu deren Empfang und Verarbeitung die Steuereinheit eingerichtet ist, um darauf basierend die Strahlungsquelle zur Emission von UV-Strahlung einzuschalten, die Messsignale des Temperatursensors und/oder des Beschleunigungssensors und/oder des Positionssensors oder eine aus einem oder mehreren dieser Messsignale abgeleitete Information umfasst.

Das Gerät kann ein in der Prozess- oder Laboranalytik verwendetes Gerät sein, z.B. ein Messgerät, ein Gerät zur Durchführung eines Liquid Handling Verfahrens oder ein Gerät zur Präparation von Proben, die biologisches Material beinhalten. Das Gerät kann beispielsweise eine Laborwaage, ein Spektrometer, ein Inkubator, ein Liquid Handling System mit einer integrierten Transportvorrichtung, ein Thermocycler, z.B. ein qPCR-Thermocycler, oder eine Roboteranlage mit einer nach außen abgeschlossenen Einhausung, sein.

Das Gerät kann Mittel zum Temperieren, insbesondere Kühlen, der mindestens einen UV-Strahlungsquelle aufweisen, z.B. Peltier-Elemente, die von dem Gerät steuerbar sind. In einer vorteilhaften Ausgestaltung ist das Gerät ein Thermocycler oder ein qPCR-Thermocycler. In diesem Fall kann der Träger des UV-Moduls im Wesentlichen dieselben Abmessungen aufweisen wie eine bestimmungsgemäß in dem Thermocycler einzusetzende Mikrotiterplatte (Englischer Fachbegriff: Sample Tray) und zur Ausführung einer Dekontamination statt der Mikrotiterplatte in den Probenraum des Thermocyclers eingesetzt werden. Die mindestens eine Strahlungsquelle weist in dieser Ausgestaltung vorteilhaft eine oder mehrere, insbesondere eine Vielzahl, UV-LEDs auf. Eine Heiz- und Kühlvorrichtung des Thermocyclers, z.B. ein Temperierblock, der im Normalbetrieb zur Einstellung des für die PCR erforderlichen Temperaturprofils dient, kann zur Kühlung der mindestens einen Strahlungsquelle dienen. Beispielsweise kann der Thermocycler, insbesondere eine in den Thermocycler integrierte Logikeinheit, bei Einsetzen des UV-Moduls in den Probenraum in einen Dekontaminations-Betriebszustand versetzt werden. Der Thermocycler bzw die, insbesondere integrierte, Logikeinheit des Thermocyclers kann in dem Dekontaminations-Betriebszustand dazu eingerichtet sein, wie weiter oben beschrieben anhand von Sensor- oder Steuersignalen das Vorliegen eines sicheren Betriebszustands zu registrieren, z.B. ob eine Abdeckung oder ein Deckel des Probenraums lichtdicht verschlossen ist. Die Logikeinheit kann weiter dazu eingerichtet sein, bei Vorliegen dieser Voraussetzung die mindestens eine Information, z.B. einen Steuerbefehl, eine Authentisierungsinformation, ein Token, eine Kennung oder ein Passwort an die Steuereinheit des UV-Moduls zu übermitteln, basierend auf der die Steuereinheit des UV-Moduls die Strahlungsquelle einschaltet. Im Dekontaminations-Betriebszustand kann der Thermocycler weiter dazu eingerichtet sein, mittels der Heiz- und Kühlvorrichtung die UV-Strahlungsquelle des UV-Moduls zu kühlen.

Das UV-Modul oder das Gerät können außerdem eine Zeitschaltuhr aufweisen, anhand derer die Steuereinheit die Strahlungsquelle nach Ablauf einer vorgegebenen Zeitspanne wieder ausschaltet.

Wie bereits beschrieben, kann das UV-Modul einen Antrieb, z.B. einen Motor, umfassen, der insbesondere von der Steuereinheit steuerbar ist, sowie einen mit dem Antrieb verbundenen Manipulator aufweisen, z.B. einen Stößel oder einen Kipp-Mechanismus. Der Antrieb kann zur Bereitstellung mechanischer Funktionen innerhalb eines Innenraums des Geräts dienen, z.B. um ein Verbrauchsmodul (englischer Fachbegriff: Consumable) innerhalb des Innenraums zu manipulieren, z.B. zu bewegen, zu entriegeln oder aus einer Ursprungslage herauszuheben. Der Motor kann von einem Akkumulator des UV-Moduls oder über eine an das UV-Modul anschließbare separate Energiequelle, z.B. eine Energieversorgung des Geräts, in dem das UV-Modul eingesetzt wird, mit Energie versorgt werden.
Die Erfindung umfasst auch ein Verfahren zur Deaktivierung von biologisch kontaminierten Oberflächen in einem Innenraum eines Geräts, insbesondere zur Handhabung und/oder Untersuchung von biologisches Material umfassenden Proben. Dieses Verfahren umfasst:
- Einbringen eines mindestens eine zur Emission von UV-Strahlung ausgestaltete Strahlungsquelle aufweisenden UV-Moduls in den Innenraum;
- Empfangen mindestens einer Information durch eine Steuereinheit des UV-Moduls; und
- Verarbeiten der mindestens einen Information durch die Steuereinheit und Betreiben, insbesondere Einschalten der Strahlungsquelle zur Emission von UV-Strahlung durch die Steuereinheit.

Das Verfahren und/oder eine der im Folgenden beschriebenen Verfahrensausgestaltungen kann bzw. können mittels des voranstehend beschriebenen UV-Moduls oder mittels des voranstehend beschriebenen Systems ausgeführt werden.

Das Verfahren kann weiter das Bestrahlen der biologisch kontaminierten Oberflächen im Innenraum mit der von der Strahlungsquelle emittierten UV-Strahlung, insbesondere über einen vorgegebenen Zeitraum, beinhalten. Hierzu kann die Steuereinheit eine Zeitschaltuhr aufweisen.

Das Gerät kann eine Logikeinheit aufweisen, wobei das Verfahren weiter umfasst:
Übertragen der mindestens Information von der Logikeinheit an die Steuereinheit, wobei die mindestens eine Information einen Steuerbefehl und/oder eine Authentifizierungs-Information umfassen kann.

In einer vorteilhaften Verfahrensausgestaltung überträgt die Logikeinheit die Information nur, wenn sich das Gerät in einem vorgegebenen Betriebszustand befindet, z.B. in einem für die Deaktivierung des Innenraums sicheren Betriebszustand, in dem der Innenraum derart abgeschlossen ist, dass keine UV-Strahlung aus dem Innenraum nach außen gelangt. Handelt es sich bei der von der Logikeinheit an die Steuereinheit übertragenen mindestens einen Information um ein Sensorsignal oder einen daraus abgeleiteten Wert, kann die Information alternativ von der Logikeinheit auch in regelmäßigen Zeitintervallen an die Steuereinheit des UV-Moduls übertragen werden. In diesem Fall kann die Steuereinheit anhand der übermittelten Information prüfen, ob der vorgegebene Betriebszustand des Geräts vorliegt und in Abhängigkeit von dem Prüfungsergebnis die Strahlungsquelle betreiben bzw. einschalten.

Mindestens während eines Zeitraums, innerhalb dessen die Strahlungsquelle UV-Strahlung emittiert, kann sie mittels einer Temperiereinrichtung, die z.B. mindestens ein Peltier-Element umfassen kann, gekühlt werden. Die Kühlung kann von der Steuereinheit und/oder von einer Logikeinheit des Geräts gesteuert werden. Handelt es sich bei dem Gerät um einen Thermocycler oder qPCR-Thermocycler, kann dieser die Strahlungsquelle mittels einer in dem Thermocycler integrierten Heiz- und Kühlvorrichtung kühlen. Hierzu kann der Thermocycler ein von der Logikeinheit ausführbares Dekontaminationsprogramm ausführen.

Das UV-Modul kann mindestens einen Sensor, insbesondere einen Strahlungsdetektor, einen Bewegungssensor, einen Positionssensor, eine Kamera oder einen Temperatursensor, aufweisen, der mit der Steuereinheit verbunden ist, um dieser ein Messsignal als die mindestens eine Information zu übertragen, und wobei das Verfahren weiter das Übertragen des Messsignals des mindestens einen Sensors an die Steuereinheit umfasst. Anhand des Messsignals des Sensors kann die Steuereinheit die von der Logikeinheit des Geräts erhaltene mindestens eine Information hinsichtlich ihrer Plausibilität überprüfen. Sie kann das Sensorsignal auch als redundante Information über das Vorliegen eines betriebssicheren Zustands des zu deaktivierenden Innenraums nutzen, um eine zusätzliche Sicherheit zur Verfügung zu stellen.

Beispielsweise kann die Steuereinheit eine erste Information von der Logikeinheit empfangen und eine zweite Information von dem mindestens einen Sensor empfangen, wobei die Steuereinheit die Strahlungsquelle nur einschaltet, wenn sich aus beiden Informationen anhand vorgegebener Kriterien ergibt, dass sich das Gerät in einem vorgegebenen Betriebszustand befindet, z.B. dass der Innenraum derart abgeschlossen ist, dass keine UV-Strahlung aus dem Innenraum nach außen gelangt.

Die Erfindung wird im Folgenden anhand der in den Figuren dargestellten Ausführungsbeispiele näher erläutert. Es zeigen:
- Fig. 1: eine perspektivische Ansicht eines Thermocyclers mit einem Probenraum zur Aufnahme von biologisches Material enthaltenden Proben;
- Fig. 2: eine Schnitt-Darstellung des in Fig. 1 dargestellten Thermocyclers;
- Fig. 3a: eine schematische Darstellung eines UV-Moduls nach einem ersten Ausführungsbeispiel zur Dekontamination des Probenraums des anhand von Fig. 1 und 2 dargestellten Thermocyclers in einer Seitenansicht;
- Fig. 3b: eine schematische Darstellung des UV-Moduls nach dem ersten Ausführungsbeispiel in einer Ansicht von oben;
- Fig. 4a: eine schematische Darstellung eines UV-Moduls nach einem zweiten Ausführungsbeispiel in einer Schnitt-Darstellung; und
- Fig. 4b: eine schematische Darstellung des UV-Moduls nach dem zweiten Ausführungsbeispiel in einer Ansicht von oben.

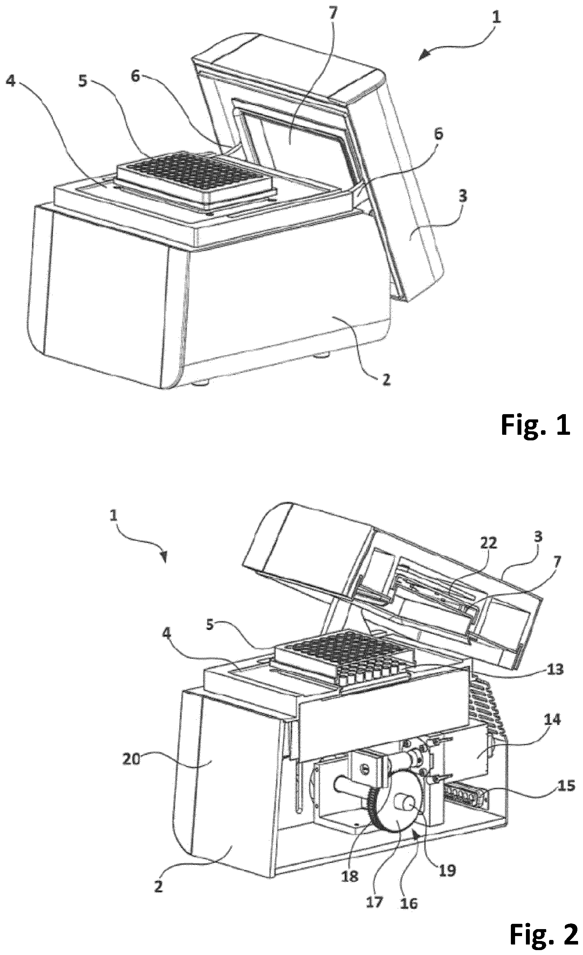

In Fig. 1 ist schematisch ein aus DE 10 2018 124 408 A1 oder DE 10 2018 124 412 A1 bekannter Thermocycler 1 zur thermischen Behandlung von Proben zur Durchführung einer Polymerasekettenreaktion (PCR) dargestellt. Die Verwendung eines erfindungsgemäßen UV-Moduls zur Deaktivierung von Oberflächen in einem Innenraum eines Geräts wird im Folgenden beispielhaft anhand dieses Thermocyclers näher erläutert. Der Thermocycler 1 besitzt eine Basiseinheit 2 und einen Deckel 3. Die Basiseinheit 2 weist einen Aufnahmebereich 4 auf, in dem sich ein Temperierblock befindet. In dem Aufnahmebereich 4 können Reaktionsgefäße angeordnet werden, in denen sich thermisch zu behandelnde Proben befinden. Im hier gezeigten Ausführungsbeispiel sind die Reaktionsgefäße in einer im Aufnahmebereich 4 angeordneten, rechteckigen Mikrotiterplatte 5 gebildet. Typischerweise bestehen solche Mikrotiterplatten aus Kunststoff. Der Deckel 3 ist über zwei Verbindungsarme 6 mit der Basiseinheit 2 verbunden. Die Verbindungsarme 6 sind mit einem in der Basiseinheit 2 angeordneten Deckelantrieb gekoppelt, der den Deckel 3 zum automatischen Öffnen und Schließen der Basiseinheit 2 bewegen kann. In dem Deckel 3 ist eine Abdeckplatte 7 angeordnet, deren zum Aufnahmebereich 4 hin weisende Vorderfläche dazu bestimmt ist, bei verschlossenem Deckel 3 gegen die in der Mikrotiterplatte 5 gebildeten Reaktionsgefäße anzuliegen. Die Abdeckplatte 7 ist im hier beschriebenen Ausführungsbeispiel beheizbar ausgestaltet. Ist der Deckel 3 geschlossen, ist zwischen der Basiseinheit 2 und dem Deckel 3 ein Probenraum gebildet, der die Probengefäße umgibt.

In Fig. 2 ist der in Fig. 1 dargestellte Thermocycler 1 in einer Schnitt-Darstellung zu sehen. Hier ist der im Gehäuse 20 der Basiseinheit 2 untergebrachte Deckelantrieb zum automatischen Verschließen des Deckels 3 und ein Temperierblock 13 zur Temperierung der Proben zu sehen. Der Deckelantrieb umfasst einen steuerbaren Motor 14, z.B. einen Elektromotor, der mit einer in der Basiseinheit 2 selbst oder außerhalb der Basiseinheit 2 angeordneten Steuerung, z.B. einer elektronische Steuerschaltung, die optional eine CPU umfassen kann, verbindbar ist. Die Steuerung dient unter anderem als Antriebssteuerung des Thermocyclers 1. Zur Verbindung des Motors 14 oder der internen Steuerung des Thermocyclers 1 mit einer geräteexternen Steuerung ist in der Basiseinheit eine Schnittstelle 15 vorgesehen. Der Deckelantrieb umfasst im vorliegenden Beispiel weiter ein von dem Motor 14 betätigbares Getriebe 16. Das Getriebe 16 weist im vorliegenden Beispiel ein Zahnrad 17 und eine schraubenförmige Schneckenwelle 18 auf, deren Drehbewegung das Zahnrad 17 in Rotation versetzt. Das Zahnrad 17 ist starr mit der Antriebswelle 19 verbunden, die über eine Führung mit den Verbindungsarmen 6 verbunden ist, um die Bewegung des Deckels 3 anzutreiben.

In der Schnittdarstellung der Fig. 2 ist auch der Aufbau des Deckels 3 im Detail zu sehen. Der Deckel 3 enthält eine mittels eines Heizmoduls beheizbare Abdeckplatte 7. Diese ist über Druckfedern mit einem Andruckblech 22 gekoppelt. Über die Druckfedern wird die vom Deckelantrieb auf die Verbindungsarme 6 und das Andruckblech 22 ausgeübte Kraft auf die Abdeckplatte 7 übertragen.

Zur abwechselnden Heizung und Kühlung der Proben für die Durchführung einer Vielzahl von PCR-Zyklen weist der Thermocycler 1 ein Temperiermodul auf, das einen Temperierblock 13 umfasst. Der Temperierblock 13 ist aus einem Metall hoher Wärmeleitfähigkeit, z.B. Silber oder Aluminium, gebildet, und weist eine Vielzahl von Aufnahmen für Reaktionsgefäße auf. Im vorliegenden Beispiel ist die Mikrotiterplatte 5 so auf den Temperierblock 13 aufgesetzt, dass die in der Mikrotiterplatte gebildeten, als Reaktionsgefäße dienenden Vertiefungen in die Aufnahmen hineinragen. Die Aufnahmen sind im hier gezeigten Beispiel als Zylinder ausgestaltet, die aufrecht auf einer Basisfläche des Temperierblocks 13 stehen. An der Unterseite des Temperierblocks 13 sind Temperierelemente, z.B. Peltier-Elemente, angeordnet, die in Kontakt mit einer Wärmesenke stehen. Die Regelung der Temperatur des Temperierblocks 13 kann ebenfalls von der Steuerung oder von einer separaten Regeleinheit ausgeführt werden.

Wie eingangs beschrieben, kann zur Deaktivierung von eventuell biologisch kontaminierten Oberflächen innerhalb des Probenraums des hier beispielhaft beschriebenen Thermocyclers ein UV-Modul eingesetzt werden, wie im Folgenden näher beschrieben wird.

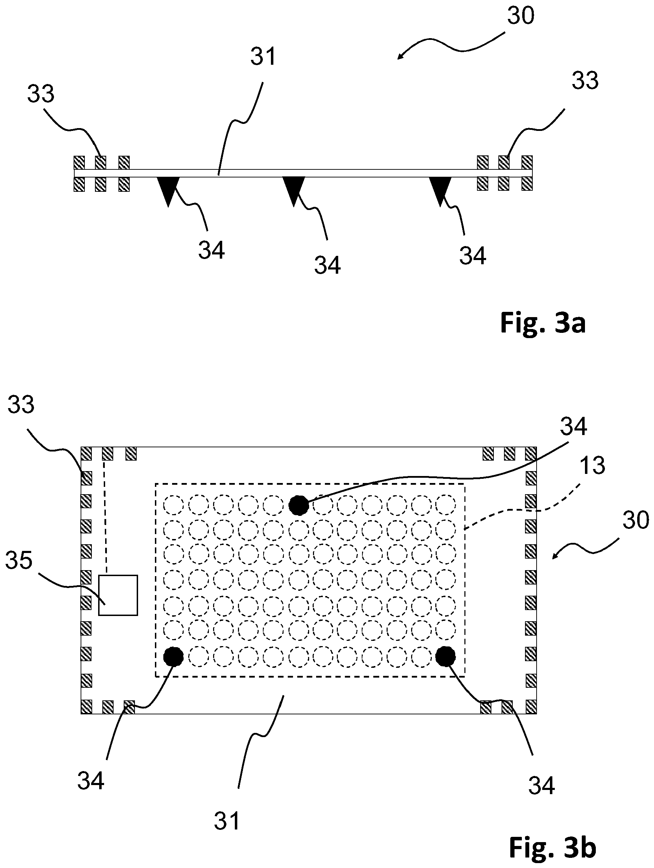

In Fig. 3a ist ein erstes Ausführungsbeispiel eines UV-Moduls 30 in einer Seitenansicht und in Fig. 3b in Draufsicht dargestellt. Es weist einen Träger 31, beispielsweise aus Kunststoff, auf, dessen geometrische Form und Abmessungen so gewählt sind, dass das UV-Modul 30 anstelle einer Mikrotiterplatte in den Probenraum des Thermocyclers 1, wie er in Fig. 1 und 2 dargestellt ist, einsetzbar ist.

Es sei hier erwähnt, dass ein ganz analog ausgestaltetes UV-Modul auch in einen Probenraum eines qPCR-Thermocyclers einsetzbar ist, da die Probenräume und Heizmodule von qPCR-Thermocyclern typischerweise ganz ähnlich und nach denselben Prinzipien ausgestaltet sind, wie die des hier in Fig. 1 und 2 dargestellten Thermocyclers 1. Zusätzlich weisen qPCR-Thermocycler Mittel zur Durchführung optischer Messungen an den in der Mikrotiterplatte enthaltenen Proben auf. Dies erfordert unter anderem, dass der Probenraum lichtdicht ausgestaltet ist. Sowohl bei dem hier dargestellten Thermocycler 1 als auch bei den herkömmlichen qPCR-Thermocyclern kann der Probenraum somit derart ausgestaltet sein, dass bei geschlossenem Deckel das Innere des Probenraums gegenüber der Umgebung derart abgeschirmt ist, dass keine UV-Strahlung aus dem Probenraum in die Umgebung gelangen kann. Das hier beschriebene UV-Modul 30 kann daher in gleicher weise in dem in Fig. 1 und 2 dargestellten Thermocycler 1 wie auch in einem abgewandelten Thermocycler, z.B. mit einem manuell betätigten Schließmechanismus, oder in einem qPCR-Thermocycler bei geschlossenem Probenraum in sicherer Weise zur Deaktivierung von Oberflächen im Inneren des Probenraums mit UV-Strahlung im UV-B oder UV-C-Bereich eingesetzt werden.

Auf dem Träger 31 sind eine Vielzahl von UV-LEDs als Strahlungsquellen 33 für die UV-Deaktivierung des Probenraums angeordnet. Die UV-LEDs sind im vorliegenden Beispiel auf zwei Seiten in einem Randbereich des Trägers 31 angeordnet und weisen in verschiedene Richtungen, so dass UV-Strahlung vom Träger 31 aus in mehrere Raumrichtungen abgestrahlt wird. Die Strahlungsquellen 33 können beispielsweise dazu ausgestaltet sein, UV-Strahlung in einem Wellenlängenbereich zwischen 250 und 280 nm zu emittieren.

Der Träger 31 weist auf einer Seite Formelemente 34 auf, die derart ausgestaltet sind, dass sie in Aufnahmen des Temperierblocks 13 (in Fig. 3b gestrichelt angedeutet) des Thermocyclers 1 passen. Diese Aufnahmen dienen im PCR-Betrieb des Thermocyclers 1 dazu, Reaktionsgefäße bzw. Vertiefungen der Mikrotiterplatte aufzunehmen. Mittels der Formelemente 34 ist sichergestellt, dass der Träger 31 in einer vorgegebenen, für die UV-Deaktivierung korrekten Position in dem Probenraum des Thermocyclers 1 angeordnet werden kann.

Auf dem Träger 31 ist außerdem eine Steuereinheit 35 angeordnet, die dazu eingerichtet ist, die Strahlungsquellen 33 zur Emission von UV-Strahlung zu steuern, z.B. einzuschalten oder auszuschalten. Die Verbindung der Steuereinheit 35 mit den Strahlungsquellen 33 ist in Fig. 3b der Übersichtlichkeit halber nur mit einer einzigen gestrichelten Verbindungslinie zwischen der Steuereinheit 35 und einer der Strahlungsquellen 33 dargestellt. Die Steuereinheit 35 kann beispielsweise eine Logikeinheit, z.B. eine Logikschaltung oder eine komplexere Recheneinheit, z.B. einen Mikrocontroller, umfassen. Sie kann außerdem eine Treiberschaltung für die Strahlungsquellen 33 enthalten. Die Treiberschaltung kann aber auch separat von der Steuereinheit 35 ausgebildet sein und von dieser gesteuert werden.

Um sicherzustellen, dass die Strahlungsquellen 33 nicht unbeabsichtigt oder missbräuchlich zur Emission von UV-Strahlung angeregt werden können, z.B. wenn das UV-Modul 30 noch nicht korrekt in dem Probenraum platziert ist, kann die Treiberschaltung von ihrer Stromquelle galvanisch getrennt sein. Diese Trennung wird von der Steuereinheit 35 nur aufgehoben, wenn sie mindestens eine Information übermittelt bekommt, die der Steuereinheit 35 anzeigt, dass der Betrieb der Strahlungsquellen 33 sicher ist, d.h. dass der Thermocycler 1 in einem für die Deaktivierung der Oberflächen innerhalb des Probenraum sicheren Betriebszustand ist. Diese mindestens eine Information kann von einem Sensor und/oder von dem Thermocycler 1 kommen.

Nach einer Ausgestaltung der hier beschriebenen Erfindung kann die in der Basiseinheit 2 oder außerhalb der Basiseinheit 2 angeordnete Steuerung des Thermocyclers 1 eine Logikeinheit bilden oder umfassen, die dazu dient, die mindestens eine Information an die Steuereinheit 35. Die Logikeinheit des Thermocyclers 1 kann z.B. eine auf einer Platine angeordnete Schaltung umfassen. Sie kann eine Logikschaltung auf Basis boolescher Algebra oder eine komplexere Recheneinheit aufweisen. Im zweiten Fall kann sie mindestens einen Prozessor, Speicherelemente und ein oder mehrere Betriebsprogramme, die in einem oder mehreren der Speicherelemente abgelegt und vom Prozessor ausführbar sind, aufweisen.

Beispielsweise kann die Logikeinheit des Thermocyclers 1 dazu eingerichtet sein, die mindestens eine Information nur an die Steuereinheit 35 zu senden, wenn sie anhand ihrer internen Steuerdaten für den Deckelantrieb oder anhand von Messsignalen eines Sensors, der die Schließung des Deckels 3 überwacht, registriert, dass der Probenraum derart geschlossen ist, dass keine UV-Strahlung aus dem Probenraum nach außen dringen kann. Ein geeigneter Sensor zur Überwachung des Schließzustands des Deckels kann z.B. ein Mikrotaster oder ein Näherungsschalter sein. Die Logikeinheit kann als die mindestens eine Information einen einfachen Steuerbefehl an die Steuereinheit 35 senden, auf dem basierend die Steuereinheit 35 die Strahlungsquellen 33 einschaltet. Es ist auch möglich, dass die Logikeinheit eine Information zur Authentisierung, ein Passwort, eine Kennung, oder ein Security Token an die Steuereinheit 35 übermittelt.

Zusätzlich oder alternativ kann das UV-Modul 30 über einen Strahlungsdetektor, z.B. einen Tageslichtsensor, z.B. eine Fotodiode oder einen Fotowiderstand, verfügen, der dazu eingerichtet ist, ein Messsignal oder eine daraus abgeleitete Größe an die Steuereinheit 35 zu kommunizieren. Anhand eines Vergleichs des Sensorsignals mit einem Soll-Signal oder einem Soll-Messwert, das bzw. der das Messsignal des Strahlungsdetektors in dem vollständig geschlossenen Probenraum repräsentiert, kann eine Sensorschaltung des Sensors oder die Steuereinheit 35 ermitteln, ob das Einschalten der UV-Strahlungsquellen sicher ist. Ist die Bedingung erfüllt, dass das Messsignal oder der Messwert des Strahlungsdetektors mit dem Soll-Signal oder Sollwert unter Berücksichtigung eines Toleranzbereichs übereinstimmt, kann die Steuereinheit 35 die Strahlungsquellen 33 zur Emission von UV-Strahlung einschalten. Anstelle eines Strahlungsdetektors können auch andere Sensoren zum selben Zweck verwendet werden, soweit ihr Messsignal einen Rückschluss darauf zulässt, ob der Probenraum ordnungsgemäß geschlossen ist.

Es ist auch möglich, dass die Steuereinheit 35 zwei oder mehr redundante Informationen von der Logikeinheit des Thermocyclers 1 und aus mindestens einer weiteren Quelle, z.B. von dem bereits erwähnten Strahlungsdetektor, erhält. Die Steuereinheit 35 ist in diesem Fall dazu eingerichtet, die Strahlungsquellen 33 nur einzuschalten, wenn beide Informationen vorliegen und einem sicheren Betriebszustand des Thermocyclers, d.h. im vorliegenden Fall einem vollständig geschlossenen Probenraum, aus dem keine UV-Strahlung nach außen dringen kann, entsprechen.

Zur Übertragung der mindestens einen Information von der Logikeinheit des Thermocyclers 1 zur Steuereinheit 35 kann ein Signal nach einem Bluetooth-, NFC- oder RFID-Standard dienen. Überträgt die Logikeinheit an die Steuereinheit 35 einen Schlüssel oder Token, um zu signalisieren, dass ein sicherer Betriebszustand vorliegt, kann dieser auch ein mechanischer oder ein elektronischer Schlüssel sein.

Für den im vorliegenden Ausführungsbeispiel beschriebenen Anwendungsfall ist eine Übertragung mittels NFC besonders günstig, da das UV-Modul nur wenige Millimeter von der Wand des Probenraums beabstandet angeordnet ist, so dass die kurzreichweitige NFC-Technik gut geeignet für die Informationsübertragung ist. Diese kurze Reichweite ist auch von Vorteil, um einen Eingriff von außen in die Kommunikation zwischen der Logikeinheit des Thermocyclers 1 und der Steuereinheit 35 zu verhindern. Alternativ kann eine Kommunikation zwischen der Logikeinheit des Thermocyclers 1 und der Steuereinheit 35 oder auch eine Kommunikation zwischen dem erwähnten Sensor und der Steuereinheit 35 leitungsgebunden, z.B. über eine Kabelverbindung oder eine flexible Leiterkarte, erfolgen.

Zur Energieversorgung kann das UV-Modul 30 einen, insbesondere kontaktlos aufladbaren, Akkumulator aufweisen (hier nicht eingezeichnet). Alternativ kann es auch mittels eines Kabels oder einer flexiblen Leiterkarte an eine Energieversorgung des Thermocyclers 1 anschließbar sein, um von diesem mit Energie versorgt zu werden.

Zur Wärmeabführung von den Strahlungsquellen 33 kann das UV-Modul 30 Kühlelemente, z.B. Peltier-Elemente, aufweisen. Im vorliegenden Beispiel wird die Kühlung jedoch mittels der Temperierelemente des Thermocyclers 1 bewerkstelligt. Dies kann in der folgenden Weise erfolgen: Soll eine Deaktivierung des Probenraums durchgeführt werden, wird der Thermocycler 1, z.B. durch eine Eingabe einer Bedienperson oder durch eine Robotersteuerung, in einen Dekontaminationsmodus versetzt und das UV-Modul 30 in den Probenraum anstelle einer Mikrotiterplatte mit Proben eingesetzt. In dem Dekontaminationsmodus prüft die Logikeinheit des Thermocyclers 1 zunächst wie weiter oben beschrieben, ob das Gerät in einem sicheren Betriebszustand ist, d.h. dass der Deckel 3 geschlossen ist, so dass keine UV-Strahlung aus dem Probenraum nach außen dringen kann. Dann sendet die Logikeinheit die mindestens eine Information an die Steuereinheit 35, die die Steuereinheit 35 verarbeitet und auf der basierend die Steuereinheit 35 die Strahlungsquellen 33 einschaltet. Es kann eine Zeitschaltuhr vorgesehen sein, die den Betrieb der Strahlungsquellen 33 für eine vorgebbare Zeitspanne aufrechterhält und nach Ablauf der Zeitspanne wieder abschaltet. Diese kann in der Steuereinheit 35 integriert sein. Mit dem Senden der Information an die Steuereinheit 35 beginnt die Logikeinheit des Thermocyclers 1 die Ausführung eines Steuerprogramms, mit dem mittels der Temperierelemente des Thermocyclers 1 eine Kühlung der Strahlungsquellen 33, insbesondere durch Einregeln einer vorgegebenen Temperatur des Temperierblocks 13, durchgeführt wird. Vorteilhaft kann das UV-Modul Wärmekontaktflächen aufweisen, die direkt und mit mechanischem Andruck gegen den Temperierblock anliegen (nicht in Fig. 3a oder 3b dargestellt).

Die Anordnung der UV-LEDs als Strahlungsquellen 33 ist in dem hier beschriebenen Ausführungsbeispiel und in Fig. 3a und 3b nur beispielhaft dargestellt. Sie kann, ebenso wie die Form und Abmessungen des Trägers 31, je nach Geometrie des Probenraums und der Leistung der LEDs sowohl zentral als auch marginal so verändert werden, dass die Innenflächen des Raumes hinreichend für die Deaktivierung ausgeleuchtet werden. Zur Einstellung einer geeigneten Strahlungsdosis kann eine Zeitschaltuhr genutzt werden.

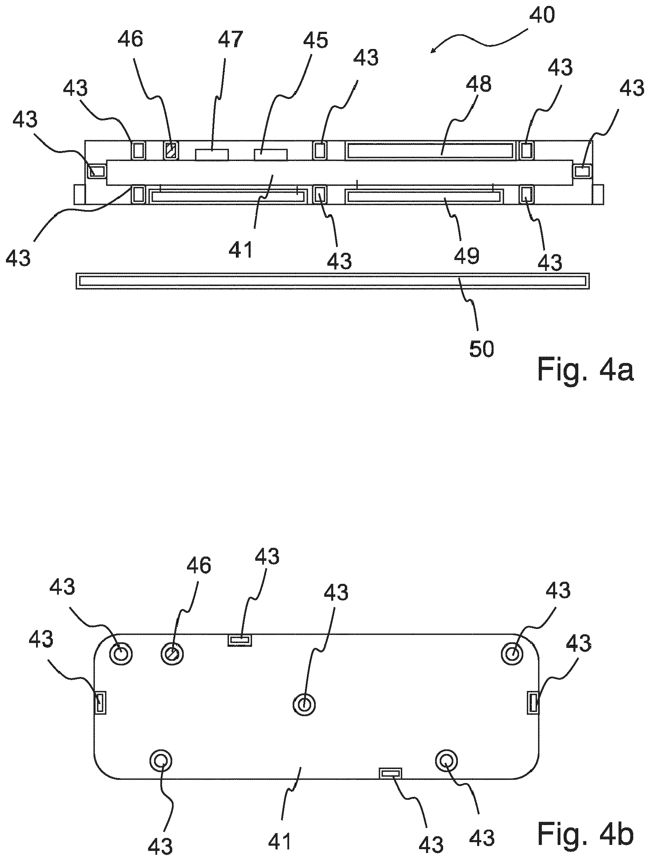

In Fig. 4a ist ein zweites Ausführungsbeispiel eines zur Deaktivierung der Oberflächen eines Innenraums in einem Gerät, z.B. einem Laborgerät zur Untersuchung oder Behandlung von biologisches Material enthaltenden Proben, geeigneten UV-Moduls 40 in einer Längsschnittdarstellung und in Fig. 4b in Draufsicht dargestellt. Das UV-Modul 40 ist in einen Innenraum eines Geräts einbringbar, um die Oberflächen innerhalb dieses Innenraums zu deaktivieren. Es weist einen Träger 41 auf, der im hier gezeigten Ausführungsbeispiel durch eine Leiterkarte gebildet ist. Auf dem Träger 41 sind eine Vielzahl von Strahlungsquellen 43 angeordnet, die im vorliegenden Beispiel als UV-LEDs ausgebildet sind. Die UV-LEDs sind sowohl an den Seiten des Trägers 41 als auch an der Ober- und Unterseite des Trägers 41 angeordnet, so dass sie in mehrere Raumrichtungen strahlen, wenn sie zur Emission von UV-Strahlung angeregt werden.

Das UV-Modul 40 weist weiter einen Prozessor auf, der als Steuereinheit 45 des UV-Moduls dient. Die Steuereinheit 45 dient unter anderem zur Anregung der UV-LEDs zur Emission von Strahlung. Weiter sind auf dem Träger 41 eine Fotodiode 46 und ein Multisensor 47 angeordnet, die mit der Steuereinheit 45 verbunden sind, um dieser Messsignale zukommen zu lassen. Der Multisensor 47 kann einen Temperatur-, Beschleunigungs- und/oder Positionssensor umfassen. Die Steuereinheit 45 ist dazu eingerichtet, die Messsignale der Fotodiode 46 und des Multisensors 47 zu verarbeiten. Mindestens eines der Messsignale kann von der Steuereinheit 45 verwendet werden, um anhand des mindestens einen Messsignals die Strahlungsquellen 43 zur Emission von Strahlung zu steuern, insbesondere einzuschalten.

Das UV-Modul 40 weist außerdem eine auf dem Träger 41 angeordnete Empfängerspule 49 auf, die als Empfängerschnittstelle dient, über die die Steuereinheit 45 Informationen mittels RFID oder NFC erhalten kann. Beispielsweise kann die Empfängerspule 49 zur Kommunikation mit einer Sendespule 50 gekoppelt werden, die in einer Wand des zu dekontaminierenden Innenraums des Geräts angeordnet ist, in dem das UV-Modul 40 eingesetzt wird. Über die durch die Empfängerspule 49 gebildete Schnittstelle kann die Steuereinheit 45 mindestens eine Information erhalten, anhand derer die Steuereinheit die Strahlungsquellen 43 zur Emission von UV-Strahlung anregen kann.

Der Träger 41 ist in einen Rahmen oder einen Verguss eingebettet, so dass die Oberfläche des UV-Moduls spaltfrei und glatt und somit gut durch Wischdesinfektion reinigbar ist.

Als Sicherheitsmaßnahme zum Verhindern eines unabsichtlichen oder unautorisierten Einschaltens kann die Steuereinheit 45 in ganz analoger Weise wie anhand des voranstehend beschriebenen Ausführungsbeispiels dazu eingerichtet sein, erst nach dem Erhalt einer Information, die einen sicheren Betriebszustand des Geräts anzeigt, die Strahlungsquellen 43 anzuschalten. Diese Information kann die Steuereinheit 45 von dem Gerät über die Empfängerspule 49 erhalten.

Die Fotodiode 46 oder der Multisensor 47 können zusätzlich oder alternativ in das Sicherheitskonzept einbezogen werden, indem sie zusätzliche Informationen liefern, anhand derer die Verlässlichkeit der über die Empfängerspule 49 erhaltenen Informationen geprüft werden kann. Dies kann im Sinne einer Mehrfaktorauthentifizierung durchgeführt werden, indem Informationen aus mehreren Quellen genutzt werden, um den Betrieb der Strahlungsquellen 43 freizugeben.

Das UV-Modul 40 umfasst außerdem einen an dem Träger 41 angeordneten, berührungslos aufladbaren Akkumulator 48. In einer automatisierten Laboranwendung ist es möglich, das UV-Modul 40, wenn es nicht benötigt wird, auf eine Ladestation aufzulegen, um den Akkumulator zu laden. Zur Dekontamination eines Laborgeräts kann der Roboter das UV-Modul 40 von der Ladestation abheben und in den zu dekontaminierenden Innenraum einsetzen. Nach der abgeschlossenen Dekontamination kann das UV-Modul 40 zurück zur Ladestation gebracht werden.

Die Erfindung wurde ausführlich anhand der Deaktivierung eines Probenraums eines Thermocyclers beschrieben. Sie kann jedoch gleichermaßen und in ganz analoger Weise auch in anderen Geräten eingesetzt werden. Auch in Geräteinnenräumen, die nicht vollständig lichtdicht gegenüber der Umgebung abgeschlossen sind, kann die Deaktivierung mittels UV-Strahlung in sicherer Weise durchgeführt werden, wenn das den Innenraum umgebende Gehäuse aus einem Material besteht, das für UV-C oder sogar für UV-B-Strahlung nicht mehr transparent ist. Dies ist z.B. bei Polycarbonat-Verbundsicherheits-Scheiben der Fall. So kann ein UV-Modul wie das in Fig. 4a und 4b gezeigte in einer Laborwaage mit Probenraum, in einem Spektrometer mit Probenraum, in einem Inkubator, in einem Liquid Handling System mit Schutzhauben und integrierten Transportvorrichtungen oder in einem Automatiklager eingesetzt werden. Eine Logikeinheit eines solchen Geräts kann in ganz entsprechender Weise wie zuvor für einen Thermocycler beschrieben, dazu eingerichtet sein, einem UV-Modul eine Information zu übermitteln, die einen sicheren Betriebszustand des Geräts, z.B. den geschlossenen Zustand des Innenraum oder Probenraums, repräsentiert.

Eine Informationsübermittlung von einem Gerät an die Steuereinheit 45 kann in einem weiteren Ausführungsbeispiel statt über eine Datenschnittstelle per Funk, NFC oder RFID auch über ein optisches Übertragungsverfahren, z.B. mittels on-line-of-sight Datenübertragung, z.B. mittels einer modulierten roten LED an die Fotodiode 46 erfolgen, die ein Signal an die Steuereinheit 45 ausgibt. UV-Module können angepasst an die konkrete Ausführung von Probenräumen oder Lagerräumen ausgeführt werden und mit automatischen Lift-, Carriage-, Stacker- und Robotersysteme transportiert werden. Hierfür kann ihre Geometrie an alternative Laborgebinde, z.B. Racks oder Disposables, z.B. Petrischalen, angepasst werden.

In automatisierten Anwendungen kann das UV-Modul durch eine horizontale Bewegung in den zu deaktivierenden Innenraum eingebracht werden. Hierbei kann auch eine Deaktivierung entlang des Fahrwegs des UV-Moduls erreicht werden. Um auch beabstandete oder durch Einbauten abgeschattete Flächen zu erreichen, kann das UV-Modul innerhalb des Innenraums verfahren werden.

In einer weiteren möglichen Ausgestaltung kann das UV-Modul 40 zusätzlich eine mechanische Funktionalität zur Verfügung stellen, insbesondere zusätzlich zur voranstehend beschriebenen Deaktivierungs-Funktionalität. Hierzu kann es einen auf dem Träger 41 befindlichen Antrieb, z.B. einen Motor und mindestens einen mit dem Motor in Wirkverbindung stehenden Manipulator aufweisen, z.B. einen Stößel oder einen Kipp-Mechanismus. Beispielsweise kann das UV-Modul 40 in ein Gerät oder eines der oben genannten abgeschlossenen Robotersysteme oder eine abgeschlossene Anlage eingesetzt werden. So kann es die zusätzliche Aufgabe übernehmen, ein sich im Innenraum befindliches Verbrauchsmodul (Fachbegriff: Consumable), z.B. eine Kartusche, durch Aktivierung des Antriebs mit angeschlossener Mechanik aus einer Ursprungslage innerhalb des Innenraums zu entfernen.

Ein Beispiel hierfür ist die Anwendung in einem Probenraum für ein Verfahren, z.B. digitale PCR, das in einer Kartusche abläuft. Hier kann das in den Innenraum eingebrachte UV-Modul dazu dienen, den Probenraum und/oder eine darin angeordnete Kartusche in der voranstehend beschriebenen Weise durch Bestrahlung zu deaktivieren und die Kartusche nach der erfolgten Deaktivierung mittels des Antriebs durch mechanische Einwirkung aus einer Verriegelung zu lösen und/oder aus einer Ursprungslage zu entfernen.

## Patentansprüche

1. UV-Modul (30, 40) zur Deaktivierung von biologisch kontaminierten Oberflächen in einem Innenraum eines Geräts, umfassend:
- einen Träger (31, 41),
- mindestens eine an dem Träger (31, 41) befestigte, zur Emission von UV-Strahlung ausgestaltete Strahlungsquelle (33, 43), und
- eine mit der mindestens einen Strahlungsquelle (33, 43) verbundene Steuereinheit (35, 45),
wobei die Steuereinheit (35, 45) dazu eingerichtet ist, mindestens eine Information zu empfangen und zu verarbeiten, um basierend auf der mindestens einen Information die Strahlungsquelle (33, 43) zur Emission von UV-Strahlung zu betreiben, insbesondere einzuschalten.

2. UV-Modul nach Anspruch 1,
weiter umfassend mindestens einen Sensor, insbesondere einen Strahlungsdetektor, einen Bewegungssensor, einen Positionssensor, eine Kamera, oder einen Temperatursensor, wobei der Sensor mit der Steuereinheit verbunden ist, um dieser ein Messsignal als die mindestens eine Information zu übertragen.

3. UV-Modul nach Anspruch 1 oder 2,
weiter umfassend einen, insbesondere berührungslos aufladbaren, Akkumulator, der mit der Steuereinheit und/oder dem mindestens einen Sensor als Spannungsquelle verbunden ist.

4. UV-Modul nach einem der Ansprüche 1 bis 3,
wobei die Steuereinheit eine Schnittstelle zur kontaktlosen Kommunikation, insbesondere nach einem Bluetooth-, NFC-, RFID- oder einem Funk-Standard, oder Mittel zur optischen Kommunikation, z.B. eine modulierbare Lichtquelle und einen Lichtempfänger, aufweist.

5. System, umfassend:
- ein Gerät, insbesondere zur Handhabung und/oder Untersuchung von biologisches Material umfassenden Proben, mit einem Innenraum; und
- ein in den Innenraum einbringbares UV-Modul nach einem der Ansprüche 1 bis 4 zur Deaktivierung von biologisch kontaminierten Oberflächen in dem Innenraum.

6. System nach Anspruch 5,
wobei das Gerät eine Logikeinheit aufweist, die dazu eingerichtet ist, die mindestens eine Information an die Steuereinheit, beispielsweise in Form eines Signals, insbesondere eines Digitalsignals, zu übertragen.

7. System nach Anspruch 6,
wobei die Logikeinheit dazu eingerichtet ist, die mindestens eine Information an die Steuereinheit zu übertragen, wenn sich das Gerät in einem vorgegebenen Betriebszustand befindet.

8. System nach Anspruch 6 oder 7,
wobei die Logikeinheit und die Steuereinheit zur drahtlosen Kommunikation, insbesondere nach einem Bluetooth-, NFC-, RFID-Standard, mittels Funk oder mittels einer optischen Kommunikation, miteinander verbunden sind.

9. System nach einem der Ansprüche 6 bis 8,
wobei die Steuereinheit dazu eingerichtet ist, die Logikeinheit anhand der mindestens einen Information zu authentifizieren, und erst nach erfolgreicher Authentifizierung die Strahlungsquelle zur Emission von UV-Strahlung einzuschalten.

10. System nach einem der Ansprüche 6 bis 9,
wobei die Logikeinheit dazu eingerichtet ist, die Steuereinheit zu authentifizieren, und erst nach erfolgreicher Authentifizierung die mindestens eine Information an die Steuereinheit zu übertragen.

11. System nach einem der Ansprüche 5 bis 10,
wobei der Innenraum dazu ausgestaltet ist, nach dem Einbringen oder durch das Einbringen des UV-Moduls verschlossen zu werden, derart dass keine UV-Strahlung aus dem Innenraum nach außen dringen kann.

12. System nach Anspruch 11,
wobei das System einen, insbesondere an dem UV-Modul angeordneten, Sensor umfasst, der dazu eingerichtet ist, ein Messsignal bereitzustellen, anhand dessen ermittelbar ist, ob der Innenraum verschlossen ist.

13. System nach Anspruch 12,
wobei die mindestens eine Information, zu deren Empfang und Verarbeitung die Steuereinheit eingerichtet ist, um darauf basierend die Strahlungsquelle zur Emission von UV-Strahlung einzuschalten, das Messsignal oder eine aus dem Messsignal abgeleitete Information umfasst.

14. System nach einem der Ansprüche 1 bis 13,
wobei das UV-Modul einen Temperatursensor und/oder einen Beschleunigungssensor und/oder einen Positionssensor und/oder eine Kamera aufweist, und wobei die mindestens eine Information, zu deren Empfang und Verarbeitung die Steuereinheit eingerichtet ist, um darauf basierend die Strahlungsquelle zur Emission von UV-Strahlung einzuschalten, die Messsignale des Temperatursensors und/oder des Beschleunigungssensors und/oder des Positionssensors oder eine aus einem oder mehreren dieser Messsignale abgeleitete Information umfasst.

15. System nach einem der Ansprüche 1 bis 14,
wobei das Gerät eine Laborwaage, ein Spektrometer, ein Inkubator, ein Liquid Handling System mit einer integrierten Transportvorrichtung, ein Thermocycler oder eine Roboteranlage mit einer nach außen abgeschlossenen Einhausung ist.

16. Verfahren zur Deaktivierung von biologisch kontaminierten Oberflächen in einem Innenraum eines Geräts, insbesondere zur Handhabung und/oder Untersuchung von biologisches Material umfassenden Proben, umfassend:
- Einbringen eines mindestens eine zur Emission von UV-Strahlung ausgestaltete Strahlungsquelle aufweisenden UV-Moduls in den Innenraum;
- Empfangen mindestens einer Information durch eine Steuereinheit des UV-Moduls; und
- Verarbeiten der mindestens einen Information durch die Steuereinheit und Betreiben, insbesondere Einschalten der Strahlungsquelle zur Emission von UV-Strahlung durch die Steuereinheit.

17. Verfahren nach Anspruch 16,
wobei das Gerät eine Logikeinheit aufweist, und wobei das Verfahren weiter umfasst:
Übertragen der mindestens einen Information von der Logikeinheit an die Steuereinheit, wobei die mindestens eine Information einen Steuerbefehl und/oder eine Authentifizierungs-Information umfasst.

18. Verfahren nach Anspruch 17,
wobei die Logikeinheit die mindestens eine Information nur überträgt, wenn sich das Gerät in einem vorgegebenen Betriebszustand befindet.

19. Verfahren nach einem der Ansprüche 16 bis 18,
wobei das UV-Modul mindestens einen Sensor, insbesondere einen Strahlungsdetektor, einen Bewegungssensor, einen Positionssensor oder einen Temperatursensor, aufweist, der mit der Steuereinheit verbunden ist, um dieser ein Messsignal als die mindestens eine Information zu übertragen, und wobei das Verfahren weiter umfasst:
Übertragen des Messsignals des mindestens einen Sensors an die Steuereinheit.

20. Verfahren nach Anspruch 19,
wobei die Steuereinheit eine erste Information von der Logikeinheit empfängt und eine zweite Information von dem mindestens einen Sensor empfängt, und wobei die Steuereinheit die Strahlungsquelle nur einschaltet, wenn sich aus beiden Informationen anhand vorgegebener Kriterien ergibt, dass sich das Gerät in einem vorgegebenen Betriebszustand befindet.
